(19) Europäisches Patentamt
European Patent Office
Office européen des brevets

(11) Veröffentlichungsnummer: **0 020 952**
**B1**

(12) # EUROPÄISCHE PATENTSCHRIFT

(45) Veröffentlichungstag der Patentschrift: **10.11.82**

(51) Int. Cl.³: **C 07 D 313/04**

(21) Anmeldenummer: **80102425.8**

(22) Anmeldetag: **05.05.80**

(54) **Verfahren zur Herstellung von Epsilon-Caprolacton aus Cyclohexanon und Perpropionsäure.**

(30) Priorität: **19.05.79 DE 2920436**

(43) Veröffentlichungstag der Anmeldung: **07.01.81 Patentblatt 81/1**

(45) Bekanntmachung des Hinweises auf die Patenterteilung: **10.11.82 Patentblatt 82/45**

(84) Benannte Vertragsstaaten: **DE FR GB NL SE**

(56) Entgegenhaltungen:
**AT-B-262 954**
**GB-A-1 203 752**

(73) Patentinhaber: **BAYER AG, Zentralbereich Patente, Marken und Lizenzen, D-5090 Leverkusen 1, Bayerwerk (DE)**
Patentinhaber: **Degussa Aktiengesellschaft, Weissfrauenstrasse 9, D-6000 Frankfurt am Main 1 (DE)**

(72) Erfinder: **Hofen, Willi, Südring 54, D-6458 Rodenbach-1 (DE)**
Erfinder: **Klenk, Herbert, Dr., Grünaustrasse 19, D-6450 Hanau-9 (DE)**
Erfinder: **Schreyer, Gerd, Dr., Wildaustrasse 22, D-6450 Hanau-9 (DE)**
Erfinder: **Weiberg, Otto, Dr., Seewiesenring 38, D-6454 Bruchköbel (DE)**
Erfinder: **Waldmann, Helmut, Dr., Carl-Rumpff-Strasse 59, D-5090 Leverkusen-1 (DE)**
Erfinder: **Seifert, Hermann, Dr., Ruwergasse 4, D-5000 Köln-80 (DE)**
Erfinder: **Reissinger, Karl-Hermann, Ottweilerstrasse 21, D-5090 Leverkusen-1 (DE)**
Erfinder: **Swodenk, Wolfgang, Dr., Auf dem Broich 5, D-5068 Odenthal-Glöbusch (DE)**

EP 0 020 952 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Epsilon-Caprolacton aus Cyclohexanon und Perpropionsäure

Die vorliegende Erfindung betrifft ein kontinuierliches Verfahren zur technischen Herstellung von ε-Caprolacton. Insbesondere betrifft die vorliegende Erfindung ein Verfahren zur Herstellung von ε-Caprolacton durch Umsetzung von organischen Lösungen von Perpropionsäure mit Cyclohexanon.

ε-Caprolacton wie auch seine Alkylderivate sind wichtige technische Produkte, die durch Polymerisation oder Copolymerisation, beispielsweise mit Epoxiden, zu hochmolekularen Produkten verarbeitet werden können, die sowohl auf dem Gebiet der Kunstharze als auch auf dem Gebiet der Schaumstoffe Anwendung finden. Daneben ist ε-Caprolacton selbst ein geeignetes Ausgangsprodukt für das auf dem Gebiet der Kunstfasern bedeutende ε-Caprolactam.

Entsprechend der grossen technischen Bedeutung dieses Produktes hat es in der Vergangenheit nicht an zahlreichen Versuchen gefehlt, einfache und wirtschaftlich günstige Verfahren zur Herstellung von ε-Caprolacton aufzufinden, die es gestatten, diese Verbindung in technischem Massstab unter Vermeidung von Koppelprodukten und umweltbelastenden Nebenprodukten herzustellen.

Die prinzipiellen Schwierigkeiten, die einer erfolgreichen Durchführung der als Baeyer-Villiger-Reaktion bezeichneten oxidativen Umwandlung eines cyclischen Ketons in ein Lacton entgegenstehen (Gleichung 1),

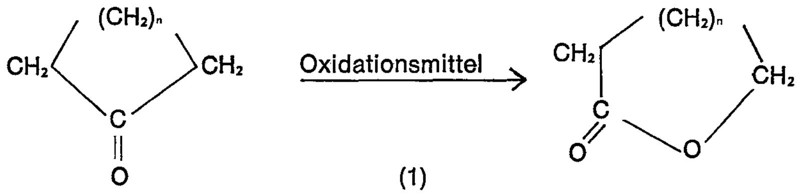


(1)

sind in der in erheblichem Umfang ablaufenden Nebenproduktbildung zu sehen. Als Nebenprodukte, die beispielsweise bei der Herstellung von ε-Caprolacton gebildet werden, sind vor allem Polyester des Lactons, polymere Peroxide, ω-Hydroxy- und ω-Acyloxy-capronsäure sowie Produkte, die aus der weiterführenden Oxidation des ε-Caprolactons herrühren, wie beispielsweise Adipinsäure, zu nennen (vgl. Houben-Weyl, Methoden der organischen Chemie, Bd. 6/2, 4. Auflage, 1963, S. 708-709 und S. 777). Für eine technische Anwendung des einen oder anderen Oxidationsmittels ist aber nun nicht nur entscheidend, welche Menge an Nebenprodukten während der Reaktion, also gleichzeitig mit dem ε-Caprolacton, gebildet wird sondern auch in welchem Umfang bei der Auftrennung der Reaktionsgemische zusätzliche Anteile an unerwünschten Produkten, insbesondere an Polymeren, auftreten.

Es ist daher versucht worden, Oxidationsmittel bzw. Lösungen von Oxidationsmitteln aufzufinden, die eine weitgehend selektive Umwandlung des Cyclohexanons zu ε-Caprolacton ermöglichen. Ebenso wurde der Versuch unternommen, Sauerstoff direkt für die Herstellung von ε-Caprolacton zu verwenden.

Nach dem Verfahren der DE-AS 1 289 840 wird Cyclohexanol mit Sauerstoff behandelt, wobei Cyclohexanon als Lösungsmittel dient. Dieses Verfahren, das über die Zwischenstufe des Cyclohexanolperoxids abläuft, arbeitet nur mit geringer Ausbeute an ε-Caprolacton, da ebenfalls Adipinsäure, 6-Hydroxycapronsäure und esterartige Kondensationsprodukte gebildet werden.

Ein Verfahren, bei dem als Oxidationsmittel ein aus einem Kohlenwasserstoff und $O_2$ gebildetes Hydroxyperoxid verwendet wird, ist aus der DE-AS 2 049 409 bekannt geworden. Die Umsetzung des Hydroperoxids mit dem cyclischen Keton erfolgt unter Verwendung von Friedel-Crafts-Katalysatoren. Der Einsatz derartiger Katalysatoren bedingt zwangsläufig einen hohen Anteil an Polymeren, da bekanntermassen durch sie eine Polymerisation der ε-Lactone ausgelöst werden kann. Zudem fällt bei diesem Verfahren als Nebenprodukt, das nicht unmittelbar wiederzurückgeführt werden kann, der dem verwendeten Hydroperoxid entsprechende Alkohol an.

Ein weiteres Verfahren, wo Sauerstoff oder ein $O_2$-haltiges Gas zur Bildung von ε-Caprolacton verwendet wird, ist der DE-AS 1 443 188 zu entnehmen. Gemäss diesem Verfahren wird die Oxidation in Gegenwart eines Aldehyds und unter Anwendung katalytischer Mengen an Metallverbindungen durchgeführt. Dieses Verfahren ist ebenso wie andere auf dieser Rohstoffbasis arbeitende Verfahren mit dem Nachteil der Koppelproduktion behaftet, da der eingesetzte Aldehyd gleichzeitig in die entsprechende Carbonsäure umgewandelt wird. Für die Herstellung von ε-Caprolacton auf dieser Rohstoffbasis muss also nicht nur ein Aldehyd zur Verfügung gestellt werden, sondern für die produzierte Carbonsäure muss auch eine geeignete Verwendung gefunden werden. Die beim Verfahren der DE-AS 1 443 188 zudem angewendeten Katalysatoren erschweren nicht nur die Aufarbeitung des ε-Caprolacton enthaltenden Reaktionsgemisches sondern fördern während der Reaktion auch die Weiteroxidation des bereits gebildeten ε-Caprolactons zu Adipinsäure und weiteren, niedermolekularen Verbindungen.

Das Verfahren der DE-AS 1 643 750 vermeidet

die Verwendung von Katalysatoren durch Einsatz von Isobutyraldehyd als Oxidationshilfsmittel. Jedoch sind auch in diesem Fall die erzielbaren Ausbeuten von 83% für eine technische Durchführung nicht ausreichend. Das grundsätzliche Problem der Koppelproduktion, bedingt durch den Anfall von Isobuttersäure, besteht auch hier.

Neben Sauerstoff ist auch $H_2O_2$ als Oxidationsmittel zur Herstellung von ε-Caprolacton herangezogen worden. Hierbei ist zur Aktivierung des Wasserstoffperoxids eine Vielzahl von anorganischen Stoffen verwendet worden, wie beispielsweise wasserfreie Fluorwasserstoffsäure [Chemical Abstracts, Vol. 47 (1953) S. 8012] oder Selensäure [Tetrahedron Letters, Bd. 13 (1959) S. 1]. Diese Verfahren können nur geringe Mengen an monomerem ε-Caprolacton liefern.

Ein Verfahren, das die gleichzeitige Herstellung von ε-Caprolacton und 6-Formyloxy-capronsäure zum Gegenstand hat, ist in der DE-AS 1 216 283 beschrieben. Bei diesem Verfahren wird als Oxidationsmittel ebenfalls $H_2O_2$ verwendet, das jedoch teilweise in Perameisensäure umgewandelt wird, bevor die Umsetzung mit dem Cyclohexanon durchgeführt wird. Neben der Notwendigkeit, in einem nachgeschalteten Verfahren die 6-Formyloxy-capronsäure in ε-Caprolacton umzuwandeln, ist weiterhin das Arbeiten mit Ameisensäure wegen der damit verbundenen Korrosionsprobleme von Nachteil.

Ein derartiges Verfahren, wo bei der Herstellung von ε-Caprolacton anfallende Nebenprodukte wie ε-Hydroxy- oder ε-Acyloxy-capronsäure sowie polymere Ester zu ε-Caprolacton aufgearbeitet werden, ist in der DE-AS 1 643 145 vorgeschlagen worden. Die genannten Nebenprodukte werden dabei mit Bor enthaltenden Verbindungen auf Temperaturen von 150-350°C erhitzt.

Der DE-OS 1 693 028 kann ein weiteres Verfahren zur Umwandlung der polymeren Ester des ε-Caprolactons entnommen werden. Diese auf einen derartigen Erfindungsgegenstand gerichteten beiden Verfahren zeigen sehr deutlich, dass die Nebenproduktbildung das beherrschende Problem bei der ε-Caprolacton-Herstellung ist.

Weiterhin ist gemäss der US-PS 2 904 584 versucht worden, Cycloalkanone mit vorher durch Umsetzung von wässrigem Wasserstoffperoxid mit Essigsäure hergestellter Peressigsäure in Gegenwart eines Kationenaustauscherharzes durch allmähliche Einführung des Cycloalkanons in das auf 70 bis 80°C gehaltene Gemisch der Persäure und des Kationenaustauschers zu oxidieren. Man erhält auf diese Weise nur ein Gemisch aus 6-Acetoxycapronsäure, 6-Hydroxycapronsäure, Adipinsäure und nur geringe Anteile an ε-Caprolacton.

In der DE-AS 1 258 858 wird vorgeschlagen, wässrige Lösungen von aliphatischen Percarbonsäuren für die Herstellung von ε-Caprolacton zu verwenden. Besonders vorteilhaft soll für diesen Zweck nach der genannten Patentschrift die Verwendung einer etwa 60 gew.%igen wässrigen Peressigsäure sein. Die im Falle des ε-Caprolactons erzielbaren Ausbeuten liegen bei 80-90%, was verglichen mit anderen Verfahren eine gewisse Verbesserung bedeutet. Die Gewinnung der wässrigen Peressigsäurelösung, die als Oxidationsmittel für dieses Verfahren zwangsläufig benötigt wird, erfordert einen hohen Aufwand an Energie, da ein mit Hilfe eines sauren Katalysators ins Gleichgewicht gebrachtes Gemisch aus $H_2O_2$, Wasser, Essigsäure und Peressigsäure der Destillation unterworfen wird, wobei nicht nur die Peressigsäure sondern auch das später in der wässrigen Lösung enthaltene Wasser destilliert werden müssen (vgl. hierzu DE-AS 2 262 970, Spalte 2, Zeile 9-15). Zudem schränken Probleme, die bei der Auswahl geeigneter Werkstoffe für die Apparate derartiger Persäure-Destillationsverfahren auftreten, und die aufwendigen Sicherheitsmassnahmen den Wert des Verfahrens der DE-AS 1 258 858 bei Anwendung in technischem Massstab erheblich ein. Darüber hinaus gestaltet sich die Aufarbeitung eines neben ε-Caprolacton noch Wasser enthaltenden Reaktionsgemisches, in dem ausserdem noch freie Carbonsäure, wie beispielsweise Essigsäure, vorliegt, wegen der unter diesen Bedingungen besonders ausgeprägten Polymerisationsneigung des Lactons äusserst schwierig und verlustreich.

Auch ist die Trennung des Wassers von der Carbonsäure, die notwendig wird, da die Carbonsäure im Kreis gefahren werden soll, aufwendig, da das Wasser abermals destilliert werden muss.

Die geschilderten Nachteile werden auch beim Verfahren der DE-AS 1 693 027 nicht überwunden, wo ebenfalls Peressigsäure und Wasser enthaltende Dampfmischungen zur Herstellung von ε-Caprolacton angewendet werden, d.h. zunächst eine wässrige Peressigsäurelösung destilliert werden muss.

Um die Schwierigkeiten, die bei Verwendung von wässrigen Percarbonsäurelösungen zur Herstellung von ε-Caprolacton aus Cyclohexanon auftreten, zu umgehen, ist gemäss Journal of the American Chemical Society, Vol. 80, 4079-82 (1958) weiterhin versucht worden, organische Lösungen von Percarbonsäuren, wie beispielsweise Peressigsäure in Aceton, zu verwenden. Jedoch auch diese Massnahme erbringt nur einen geringen Erfolg, da bei Verwendung von Peressigsäure in Aceton sehr lange Reaktionszeiten von sechs und mehr Stunden erforderlich sind, um im Falle des ε-Caprolactons Ausbeuten von 85% zu erzielen. Zudem muss die aus der Peressigsäure sich bildende Essigsäure schnell aus dem Reaktionsgemisch bzw. von ε-Caprolacton abgetrennt werden, wenn Ausbeuteverluste durch Polymerisation des Lactons vermieden werden sollen.

Es wird daher vorgeschlagen, ein weiteres Lösungsmittel anzuwenden, das, wie beispielsweise Äthylbenzol, in der Lage ist, mit Essigsäure ein Azeotrop zu bilden (a.a.O., 80, 4080 [1958]).

Die Verwendung eines derartigen Hilfsstoffes bedeutet einen zusätzlichen Aufwand.

Neben Peressigsäure wird gemäss DE-AS 1 086 686 auch das hochexplosive Acetaldehydmonoperacetat als Oxidationsmittel verwendet, um Cyclohexanon in das entsprechende Lacton zu überführen.

Peressigsäure bzw. das Acetaldehydmonoperacetat werden jeweils durch Oxidation von Acetaldehyd mit Sauerstoff hergestellt. Bekanntermassen hat jedoch diese Methode, Aldehyde durch Oxidation in einem organischen Lösungsmittel in Percarbonsäuren umzuwandeln, erhebliche Nachteile. Bei der Durchführung des Verfahrens treten einmal hochexplosive Zwischenprodukte auf (vgl. DE-AS 2 262 970), was bei der technischen Durchführung aufwendige Sicherheitseinrichtungen notwendig werden lässt. Weiterhin ist es sehr nachteilig, dass die den Aldehyden jeweils entsprechenden Carbonsäuren nach erfolgter Oxidation, beispielsweise der von Cyclohexanon zu ε-Caprolacton, als Nebenprodukte anfallen, die in diesem Falle nicht wieder in die Stufe zur Gewinnung der Lösung der Percarbonsäure eingesetzt werden können (DE-AS 1 258 858, Spalte 1, Zeile 23-31, sowie DE-AS 1 443 188, Spalte 2, Zeile 7-16).

Darüber hinaus ist zu berücksichtigen, dass die nach dem Aldehyd-Oxidations-Verfahren gewonnenen Percarbonsäurelösungen Verunreinigungen enthalten, die durch den bei der Herstellung gleichzeitig ablaufenden oxidativen Abbau des Aldehyds hervorgegangen sind und die wegen ihrer sauren Eigenschaften in der Lage sind, die unerwünschte und zu Verlusten führende Polymerisation des Lactons schon im Reaktionsgemisch auszulösen.

In der DE-OS 2 038 455 ist ein weiteres Verfahren zur Herstellung von ε-Caprolacton vorgeschlagen worden. Hierbei handelt es sich um ein aufwendiges, mehrstufiges Verfahren. Zunächst wird eine wässrige Lösung von $H_2O_2$ hergestellt durch Umsetzung von Cyclohexanol mit molekularem Sauerstoff, nachfolgender Hydrolyse des gebildeten Cyclohexanolperoxids und Extraktion des nunmehr vorliegenden $H_2O_2$ mit Wasser. Danach wird das wässrige Wasserstoffperoxid in ein Phosphor enthaltendes Lösungsmittel überführt und Wasser abdestilliert, worauf der organischen Lösung des $H_2O_2$ eine Carbonsäure und saurer Katalysator zugesetzt werden. Nach Zusatz eines Schleppmittels wird dieses zusammen mit dem Reaktionswasser der Persäure-Bildungsreaktion und der Percarbonsäure abdestilliert. Die gewonnene Persäure wird nunmehr mit dem Cyclohexanon umgesetzt. Wie dem Beispiel 1 der DE-OS 2 038 455 entnommen werden kann, wird nur eine 70%ige Ausbeute an ε-Caprolacton erhalten. Der bei Durchführung eines derartigen Verfahrens benötigte Aufwand an Apparaturen und Energien ist sehr hoch, wie unschwer aus der Zahl der Verfahrensschritte abgelesen werden kann.

Zusammenfassend kann aufgrund der den Stand der Technik darstellenden Literatur über Verfahren zur Herstellung von Lactonen festgestellt werden, dass alle bekannten Verfahren, einschliesslich der Verfahren, bei denen zur Oxidation von Cyclohexanonen Percarbonsäuren verwendet werden, eine zufriedenstellende Bewältigung der durch technische Erfordernisse und die Frage der Wirtschaftlichkeit aufgegebenen Probleme nicht zu bieten vermögen.

Demgegenüber wurde nun gefunden, dass man in einfacher, technisch sowie wirtschaftlich vorteilhafter Weise ε-Caprolacton durch Umsetzung von Cyclohexanon mit Perpropionsäure herstellen kann, wenn man

a) eine Lösung von Perpropionsäure in einem organischen Lösungsmittel, das mindestens 15°C unterhalb des Siedepunktes von Propionsäure siedet, mit Cyclohexanon im Molverhältnis Cyclohexanon : Perpropionsäure wie 1,1 - 5 : 1 bei Temperaturen von 10 - 80°C umsetzt,

b) das erhaltene, im wesentlichen aus ε-Caprolacton, Cyclohexanon, Propionsäure und organischem Lösungsmittel bestehende Reaktionsgemisch einer ersten Destillationseinheit zuführt, wo das organische Lösungsmittel für die Perpropionsäure als Destillat wiedergewonnen wird,

c) das Sumpfprodukt der ersten Destillationseinheit in eine zweite Destillationseinheit eingibt, wo die Propionsäure zusammen mit in Stufe (a) nicht umgesetztem Cyclohexanon als Kopfprodukt gewonnen wird und ε-Caprolacton sowie gegebenenfalls hochsiedende Anteile getrennt voneinander unterhalb des Einlaufes in diese zweite Destillationseinheit abgezogen werden und

d) das aus Propionsäure und Cyclohexanon bestehende Destillat der zweiten Destillationseinheit in eine dritte Destillationseinheit überführt, wo ein aus Propionsäure und Cyclohexanon bestehendes Gemisch erhalten und als Destillat Propionsäure wiedergewonnen wird.

Als Lösungsmittel für die Perpropionsäure seien alle gegenüber dieser Percarbonsäure inerten, organischen Lösungsmittel genannt.

Beispielsweise erwiesen sich als geeignet aromatische Kohlenwasserstoffe, die 6 bis 10 Kohlenstoffatome enthalten, aliphatische oder cycloaliphatische, jeweils bis zu 12 Kohlenstoffatome enthaltende Kohlenwasserstoffe, chlorierte Kohlenwasserstoffe, die 1 bis 10 Kohlenstoffatome sowie 1 bis 4 Chloratome enthalten, und Ester von 1 bis 5 C-Atomen enthaltenden Carbonsäuren mit geradkettigen oder verzweigten Alkoholen, in denen 1 bis 8 C-Atome im Molekül vorliegen, sowie Äther, die 2 bis 10 C-Atome enthalten. Beispielsweise seien als Lösungsmittel genannt: Benzol, Toluol, n-Pentan, Isooctan, Cyclohexan, Methylenchlorid, Chloroform, 1,2-Dichloräthan, 1,2-Dichlorpropan, Methylacetat, Äthylacetat, n-Propylacetat, Isopropylacetat, Methylpropionat, Äthylpropionat und Propylpropionat, sowie Äther. Es ist aber auch möglich, als Lösungsmittel für die Perpropionsäure Gemische aus den genannten Lösungsmitteln zu verwenden, wobei man dann vorteilhafterweise die Komponenten des Gemisches so wählt, dass sie

einen ähnlichen Siedepunkt besitzen. Man benutzt als organisches Lösungsmittel für die Perpropionsäure eine Verbindung, die mindestens 15°C unterhalb des Siedepunktes der Propionsäure siedet. Bevorzugt werden chlorierte Kohlenwasserstoffe, wie Methylenchlorid, Dichloräthan, 1,2-Dichlorpropan, Trichloräthylen oder Tetrachloräthylen oder aromatische Kohlenwasserstoffe, wie Benzol, oder Äther, wie Diisopropyläther und Ester, wie Essigsäure- oder Propionsäurepropylester, oder auch Gemische dieser Lösungsmittel verwendet. Ganz besonders bevorzugt wird als Lösungsmittel für das erfindungsgemässe Verfahren Benzol, 1,2-Dichlorpropan oder Tetrachloräthylen verwendet.

Die Konzentration der Perpropionsäure in der zur Umsetzung mit dem Cyclohexanon gelangenden organischen Lösung kann in weiten Grenzen schwanken. Im allgemeinen sind Konzentrationen von 3 bis 60 Gew.-% geeignet. Bevorzugt verwendet man Lösungen, die 5 bis 50 Gew.-% Perpropionsäure enthalten, ganz besonders bevorzugt solche, die einen Gehalt von 5 bis 30 Gew.-% an Perpropionsäure aufweisen.

Organische Lösungen von Perpropionsäure, die für das erfindungsgemässe Verfahren geeignet sind, können neben der Perpropionsäure noch freie Propionsäure enthalten. Die Menge an Propionsäure, die neben der Perpropionsäure vorhanden sein kann, ist für das erfindungsgemässe Verfahren nicht von Bedeutung. Sie kann grösser oder kleiner sein als die Menge an Perpropionsäure in der Lösung. Im allgemeinen wird man jedoch Lösungen mit dem Cyclohexanon zur Reaktion bringen, in denen die Menge an Propionsäure unter der der Perpropionsäure liegt. Beispielsweise beträgt der Gehalt an Propionsäure in der Perpropionsäurelösung 1 bis 50, bevorzugt 5 bis 40 Gew.-%, ganz besonders bevorzugt 5 bis 20 Gew.-%.

Es kann in einigen Fällen von Vorteil sein, die organische, weitgehend wasser- und wasserstoffperoxidfreie Lösung der Perpropionsäure mit einem Stabilisator zu versetzen. Als Stabilisatoren können Stickstoff oder Hydroxylgruppen enthaltende Carbonsäuren oder Polycarbonsäuren, aber auch Phosphorverbindungen, wie beispielsweise die Natriumsalze von partiell mit langkettigen Alkoholen veresterten Polyphosphorsäuren, verwendet werden (vgl. D. Swern «Organic Peroxides» Vol. 1, S. 350, 1. Abschnitt; Wiley-Interscience 1970). In vielen Fällen ist aber eine Stabilisierung nicht notwendig, da bei den Temperaturen, bei denen das erfindungsgemässe Verfahren durchgeführt wird, eine das Verfahren beeinträchtigende, wesentliche Zersetzung der Perpropionsäure nicht auftritt.

In technisch vorteilhafter Weise wird die organische Lösung der Perpropionsäure beispielsweise gemäss dem Verfahren der Deutschen Patentschrift 2 262 970 durch Extraktion eines durch Umsetzung von Wasserstoffperoxid, Wasser und saurem Katalysator mit der Propionsäure gewonnnenen Reaktionsgemisches mit dem inerten organischen Lösungsmittel und gegebenenfalls anschliessende Trocknung des im wesentlichen die Perpropionsäure enthaltenden Extraktes gewonnen.

Die für das erfindungsgemässe Verfahren verwendeten organischen Lösungen der Perpropionsäure enthalten im allgemeinen nicht mehr als 5 Gew.-% Wasser. Bevorzugt eignen sich Lösungen von Perpropionsäure, die weniger als 3 Gew.-% Wasser enthalten. Besonders bevorzugt gelangen Lösungen von Perpropionsäure in einem inerten organischen Lösungsmittel zur Anwendung, deren Wassergehalt unter 1 Gew.-% liegt. Ganz besonders bevorzugt sind Lösungen, die nicht mehr als 0,5 Gew.-% Wasser enthalten.

Der Gehalt an freiem Wasserstoffperoxid in den organischen Lösungen der Perpropionsäure, die für das erfindungsgemässe Verfahren geeignet sind, beträgt im allgemeinen nicht mehr als 2 Gew.-%. Bevorzugt werden Lösungen eingesetzt, die weniger als 1 Gew.-% $H_2O_2$ enthalten. Ganz besonders bevorzugt werden organische Lösungen von Perpropionsäure verwendet, die weniger als 0,25 Gew.-% Wasserstoffperoxid enthalten.

Organische Lösungen von Perpropionsäure, die für das Verfahren gemäss der Erfindung geeignet sind, enthalten im allgemeinen bei säurekatalysierter Herstellung noch geringe Anteile an saurem Katalysator. Im allgemeinen beträgt der Gehalt an freier starker Säure, wie beispielsweise Methansulfonsäure, Trifluormethansulfonsäure, Perchlorsäure, Schwefelsäure, Sulfonsäuren des Benzols oder des Naphthalins oder Trifluoressigsäure, weniger als 1 Gew.-%. Besonders geeignet sind Lösungen, deren Gehalt an starker Säure unter 0,1 Gew.-% liegt. Ganz besonders geeignet sind organische Lösungen von Perpropionsäure, die weniger als 100 ppm an starker Säure enthalten.

Die gemäss Verfahrensschritt (a) erfolgende Umsetzung der organischen Lösung der Perpropionsäure mit überschüssigem Cyclohexanon wird bei Temperaturen von 10 bis 80°C, vorzugsweise bei 20 bis 60°C durchgeführt.

Das Molverhältnis von Cyclohexanon zu Perpropionsäure beträgt bevorzugt 1,1 bis 5:1. Ganz besonders vorteilhaft ist es für die Durchführung des erfindungsgemässen Verfahrens, ein Molverhältnis von 1,5 bis 2,5 Mol Cyclohexanon je Mol eingesetzter Perpropionsäure anzuwenden.

Der Druck ist für die Reaktion nicht entscheidend. Prinzipiell kann die Reaktion bei erhöhten Drücken oder auch bei vermindertem Druck durchgeführt werden. Die Reaktionskomponenten können auch teilweise in gasförmiger Form vorliegen.

Um die Reaktionswärme abzuführen, kann man mit einem geeigneten Medium kühlen. Um die gewünschte Reaktionstemperatur exakt einzustellen, wählt man beispielsweise den Druck im Reaktionsgefäss so, dass die Reaktionsmischung gerade siedet. Bevorzugt führt man die Reaktion bei Drücken von 0,8-1,3 bar durch.

Zur Durchführung der Reaktion können die für Umsetzungen dieser Art üblichen Vorrichtungen verwendet werden, wie Rührkessel, Röhrenreaktoren oder Schlaufenreaktoren. Im allgemeinen verwendet man bei kontinuierlicher Durchführung der Reaktion eine Vorrichtung, die sich wie eine Kaskade von mindestens zwei ideal durchmischten Kesseln verhält. Besonders vorteilhaft ist es, ein Reaktionssystem zu verwenden, das sich wie eine Kaskade von 4 bis 50, vorzugsweise 5 bis 20, ideal durchmischten Kesseln verhält.

Neben der Arbeitsweise unter isothermen Bedingungen, d.h. Einhaltung einer einheitlichen Temperatur im gesamten Reaktionsgemisch, kann man die Umsetzung auch unter Ausbildung eines sogenannten Temperaturgradienten durchführen, der im allgemeinen mit fortschreitender Reaktion zunimmt. Man kann aber auch die Reaktion so führen, dass sich mit dem Fortschreiten der Reaktion ein Gradient fallender Temperatur ausbildet.

Als Werkstoffe, aus denen die Vorrichtungen zur Durchführung der Reaktion gefertigt sein können, kommen Glas, Email oder legierte Edelstähle in Frage.

Beispielsweise eignen sich Werkstoffe, die neben Eisen im wesentlichen noch Chrom und Nickel enthalten, wie beispielsweise ein Werkstoff mit der DIN-Bezeichnung 1 4571, der neben Eisen 17,5 Gew.-% Chrom, 11,5 Gew.-% Nickel, 2,25 Gew.-% Molybdän, sowie bis zu 2 Gew.-% Mangan, bis zu 1 Gew.-% Silicium, bis zu 0,1 Gew.-% Kohlenstoff und geringe Mengen Titan enthält; oder ein Werkstoff, der neben Eisen 25 Gew.-% Chrom, 25 Gew.-% Nikkel, 2,25 Gew.-% Molybdän und bis zu 2 Gew.-% Mangen, bis zu 1 Gew.-% Silicium, bis zu 0,06 Gew.-% Kohlenstoff sowie geringe Mengen Titan enthält und nach DIN mit der Nummer 1 4577 bezeichnet wird.

Die Reaktionsdauer ist abhängig von der Temperatur und der Konzentration der Perpropionsäure und des Cyclohexanons sowie der Art des Lösungsmittels, in dem die Perpropionsäure zum Einsatz gelangt. In der Regel wählt man die Reaktionsbedingungen derart, dass die Perpropionsäure nach 20 bis 150 Minuten, vorzugsweise nach 30 bis 120 Minuten zu über 99% umgesetzt ist.

Die Aufarbeitung des ε-Caprolacton enthaltenden Reaktionsgemisches erfolgt gemäss den Verfahrensstufen (b), (c) und (d) unter Anwendung mehrerer Destillationsschritte.

Für die technische Durchführung dieser Abfolge von drei Destillationsschritten kommen die bekannten Boden- oder Füllkörperkolonnen in Frage. Ebenso geeignet sind Kolonnen, die mit Packungen oder Einbauten aus Draht- oder Glasfasergewebe ausgerüstet sind. Auch Pakkungen aus keramischen Materialien können mit Erfolg eingesetzt werden. Es ist auch möglich, eine Kombination aus Boden- und Füllkörperkolonnen zu verwenden, beispielsweise derart, dass eine Kolonne im Abtriebsteil mit Pallringen aus Edelstahl bestück wird, während im Verstärkerteil derselben Kolonne Glockenböden zum Einsatz gelangen.

Jede der erfindungsgemäss verwendeten drei Destillationskolonnen ist jeweils mit einer Verdampfereinheit und einem Kondensationssystem versehen. Als Verdampfer können die bekannten Konstruktionen, wie Fallstrom-, Umlauf- oder Kletterverdampfer sowie Dünnschichtverdampfer, oder einfache Drallrohre eingesetzt werden. Umlaufverdampfer sind für das erfindungsgemässe Verfahren im allgemeinen gut geeignet. Für die Durchführung der Stufe (c) des Verfahrens werden in vorteilhafter Weise Kombinationen von Umlauf- bzw. Fallfilmverdampfer und Dünnschichtverdampfer eingesetzt. Die Verweilzeiten, denen die jeweiligen Sumpfprodukte in den Sümpfen und Verdampfereinheiten der Kolonnen unterliegen, werden durch entsprechende Dimensionierung dieser Apparate vorteilhafterweise so bemessen, dass sie weniger als 15 Minuten betragen.

Als Materialien, aus denen der gesamte Destillationsturm ebenso wie die Einbauten und die Verdampfungs- und Kondensationseinrichtungen gefertigt werden können, kommen alle im Chemieanlagenbau üblicherweise verwendeten Werkstoffe in Frage, wie beispielsweise rostfreie Edelstähle, die neben Eisen als Legierungsbestandteil im wesentlichen noch Chrom und Nikkel enthalten.

Die Anzahl der theoretischen Trennstufen, die eine im erfindungsgemässen Verfahren verwendete Destillationskolonne im allgemeinen besitzt, kann in weiten Grenzen schwanken. Im allgemeinen ist eine theoretische Trennstufenzahl von 3 bis 50 ausreichend. Für die im Verfahrensschritt (b) benutzte Kolonne richtet sich der erforderliche Trennaufwand im wesentlichen nach den Eigenschaften, die die Verbindung besitzt, die als Lösungsmittel für die Perpropionsäure dient.

Erfindungsgemäss erfolgt die Trennung der einzelnen Komponenten des die Verfahrensstufe (a) verlassenden Reaktionsgemisches in der nachstehend beschriebenen Weise (Fig. 1).

Das Reaktionsgemisch aus Stufe (a), das neben dem organischen Lösungsmittel und Propionsäure etwa 5-30 Gew.-% ε-Caprolacton und etwa 5-20 Gew.-% Cyclohexanon enthält, wird gegebenenfalls nach Abscheidung von geringen Mengen an gasförmigen Anteilen, der ersten Destillationseinheit (1) der Stufe (b) über Leitung (2) zugeführt. In dieser ersten Kolonne wird das organische Lösungsmittel für die Perpropionsäure als Kopfprodukt gewonnen. Dieses Lösungsmittel wird über Leitung (3) der Gewinnung der organischen Lösung der Perpropionsäure wiederzugeführt. Die Betriebsbedingungen in der ersten Kolonne werden so gewählt, dass das als Destillat gewonnene organische Lösungsmittel weniger als 3 Gew.-%, besonders bevorzugt, weniger als 1 Gew.-% Propionsäure enthält. Der Gehalt an organischem Lösungsmittel in dem über (2) in die erste Kolonne gelangenden Gemisch kann in weiten Grenzen schwanken und ist für das erfindungsgemässe Verfahren nicht von Bedeutung. Die Konzentration an organischem Lösungsmittel im Strom (2) ist im wesentlichen bestimmt durch den Gehalt an Perpropionsäure in der Lösung, die über (4) der Reaktionsstufe (a) zugeführt wird.

Im allgemeinen wird der ersten Destillationskolonne (1) ein Reaktionsgemisch zugeführt, in dem das Gewichtsverhältnis von Propionsäure zu Cyclohexanon 0,5 - 4 : 1, bevorzugt 1 - 3 : 1 beträgt. Ganz besonders bevorzugt wird dieses Verhältnis so gewählt dass es 1,5 - 2,5 : 1 beträgt.

Die für Verfahrensschritt (b) verwendete Destillationskolonne besitzt im allgemeinen insgesamt 2-50 theoretische Trennstufen. Bevorzugt beträgt die theoretische Trennstufenzahl im Abtreiberteil 1 - 30 und im Verstärkerteil 1 - 20.

Die erste Kolonne (1) kann bei Normaldruck oder leichtem Unterdruck betrieben werden. Es ist aber auch möglich, sie bei geringem Überdruck zu betreiben. Der bevorzugte Druckbereich liegt zwischen 0,1 und 1,5 bar. Ganz besonders vorteilhaft ist es, die Kolonne bei 0,1 - 1,0 bar zu betreiben. Der Temperaturbereich, bei dem die erste Destillationseinheit betrieben wird, richtet sich selbstverständlich nach dem für die Perpropionsäure verwendeten organischen Lösungsmittel; die Temperatur der Destillation kann dementsprechend breit variiert werden.

Das Rücklaufverhältnis, d.h. Menge an organischem Lösungsmittel in Strom (5) zu Menge in Strom (3), kann in weiten Grenzen variiert werden.

Dieses Rücklaufverhältnis richtet sich nicht nur nach den physikalischen Eigenschaften der jeweiligen Verbindung, die als organisches Lösungsmittel für die Perpropionsäure gewählt wurde, sondern auch nach der Trennstufenzahl, die die erste Kolonne besitzt. Im allgemeinen ist ein Rücklaufverhältnis von 0,5 - 5 : 1 für alle in Frage kommenden Lösungsmittel ausreichend. Bevorzugt wählt man ein Rücklaufverhältnis, das im Bereich von 0,5 - 2 : 1 liegt.

Das als Produktstrom (3) gewonnene organische Lösungsmittel kann neben geringen Mengen an Propionsäure auch geringste Anteile an Wasser enthalten, die sich je nach Dichte des Lösungsmittels gegebenenfalls als leichte oder schwere Phase abscheiden. Da der Wasseranteil sehr gering ist, tritt im allgemeinen überhaupt keine Phasentrennung auf. Ein gegebenenfalls als wässrige Phase im Kopfprodukt der Kolonne (1) anfallender Anteil kann verworfen werden oder aber der der Gewinnung der Perpropionsäure-Lösung dienenden Verfahrensstufe an geeigneter Stelle zugeführt werden.

Das Sumpfprodukt der Destillationskolonne (1), das im wesentlichen das ε-Caprolacton, die Propionsäure und das, bezogen auf Perpropionsäure, im Überschuss in Stufe (a) eingesetzte Cyclohexanon enthält, wird gemäss Verfahrensschritt (c) nunmehr über Leitung (6) einer zweiten Destillationseinheit (7) zugeführt, wo Propionsäure und Cyclohexanon gemeinsam als Kopfprodukt gewonnen werden, während ε-Caprolacton an einer Stelle aus Kolonne (7) abgezogen wird, die sich 1 bis 15 bevorzugt 3 - 10 theoretische Trennstufen oberhalb des Sumpfes befindet. Gegebenenfalls mit dem Produktstrom (6) in die Kolonne (7) in geringer Menge eingebrachte bzw. in dieser Kolonne gebildete höhersiedende Anteile, wie Adipinsäure und Poly-Caprolacton, werden als Sumpfprodukt über (8) ausgetragen. Dieser Anteil ist jedoch, verglichen mit bekannten Verfahren, beim Verfahren gemäss der Erfindung äusserst gering. Daneben muss betont werden, dass weder im Strom (6) noch im Ablauf (8) oder im Produktaustrag (9) irgenwelche Verbindungen peroxidischer Natur nachweisbar sind, was vom sicherheitstechnischen Standpunkt sehr vorteilhaft ist.

Der Einlauf des Stroms (6) in diese zweite Destillationseinheit (7) befindet sich 1 bis 20, bevorzugt 2 - 15 theoretische Trennstufen oberhalb der Stelle, wo das ε-Caprolacton im Seitenabzug (9) gewonnen wird. Der oberhalb des Einlaufs in die Kolonne (7) befindliche Kolonnenteil umfasst im allgemeinen 3 - 20 theoretische Trennstufen. Vorteilhafterweise gestaltet man diesen Teil der Kolonne so, dass die theoretische Trennstufenzahl im Bereich von 5 - 10 liegt.

Der Druck in der Kolonne (7) wird so gewählt, dass er 5 - 500 mbar beträgt. Man kann aber auch bei höheren Drücken arbeiten, was naturgemäss höhere Kopf- und Sumpftemperaturen zur Folge hat. Als besonders vorteilhaft hat es sich erwiesen, einen Bereich für den Druck, gemessen am Kopf der Kolonne, zu wählen, der zwischen 5 und 300 mbar liegt. Ganz besonders günstig ist es, die Kolonne (7) unter einem Druck zu betreiben, der, unter Berücksichtigung der Druckverluste durch die Einbauten in der Kolonne, an der Stelle, wo das ε-Caprolacton im Seitenstrom (9) entnommen wird, eine Temperatur von 110 - 140°C ergibt.

Das Rücklaufverhältnis, mit dem in der Kolonne (7) bei der vorgegebenen theoretischen Stufenzahl eine ausreichende Trennung des Gemisches von Propionsäure und Cyclohexanon gegenüber ε-Caprolacton erreicht wird, kann sehr klein gehalten werden. Im allgemeinen beträgt das Rücklaufverhältnis 0,5 - 5 : 1, häufig 0,5 - 3 : 1. Besonders vorteilhaft arbeitet man bei einem Rücklaufverhältnis von 0,5 - 1,5 : 1.

Die Zusammensetzung des Gemisches, das in Kolonne (7) aufgetrennt wird, kann in weiten Grenzen schwanken.

Beispielsweise enthält der Produktstrom (6) 10 - 40 Gew.-% ε-Caprolacton, 35 - 55 Gew.-% Propionsäure und 15 - 35 Gew.-% Cyclohexanon. Meist jedoch liegen die Konzentrationen für diese drei Verbindungen im Bereich von 20 - 35 Gew.-%, bzw. 40 - 50 Gew.-% und 20 - 30 Gew.-%.

Das als Seitenstrom aus der Kolonne (7) über (9) entnommene ε-Caprolacton besitzt eine

Reinheit von über 98 Gew.-% und ist für den grössten Teil der Anwendungszwecke bereits geeignet. Als Verunreinigungen, die im Produktstrom (9) enthalten sind, treten praktisch nur Propionsäure und Cyclohexanon auf. Beispielsweise hat das über (9) entnommene ε-Caprolacton die folgende Zusammensetzung: 98 - 99 Gew.-% ε-Caprolacton, 0,2 - 0,8 Gew.-% Cyclohexanon und 0,3 - 0,7 Gew.-% Propionsäure.

Das über (9) aus Kolonne (7) abgezogene ε-Caprolacton kann gegebenenfalls auch, wenn dies für den jeweiligen Verwendungszweck des Produktes angezeigt sein sollte, einer noch weitergehenden Reinigung, beispielsweise durch Destillation, unterworfen werden.

Als Kopfprodukt der Destillationseinheit (7) wird, wie bereits erwähnt, ein Gemisch aus Propionsäure und Cyclohexanon gewonnen, das ohne Nachteil geringe Mengen an ε-Caprolacton enthalten kann. Im allgemeinen wählt man die Destillationsbedingungen in Kolonne (7) so, dass der Gehalt an ε-Caprolacton im Destillat unter 1 Gew.-%, bevorzugt unter 0,5 Gew.-% liegt. Das Gewichtsverhältnis von Propionsäure zu Cyclohexanon im Kopfprodukt der Kolonne (7) beträgt besonders bevorzugt 1,5 - 2,5 : 1. Beispielsweise erhält man ein Gemisch, das neben 65 Gew.-% Propionsäure, 34,9 Gew.-% Cyclohexanon sowie 0,1 Gew.-% ε-Caprolacton enthält.

Das Kopfprodukt der Kolonne (7) wird nunmehr über Leitung (10) erfindungsgemäss dem Verfahrensschritt (d) zugeführt, der in der Destillationseinheit (11) vorgenommen wird. Im Verfahrensschritt (d) wird aus dem Produktstrom (10) Propionsäure als Destillat der Kolonne (11) gewonnen und über (12) der Herstellung der organischen Lösung der Perpropionsäure wiederzugeführt.

Gleichzeitig wird aus (11) an einer Stelle, die sich zwischen Einlauf des Stromes (10) in die Kolonne (11) und dem Sumpfauslass (14) befindet, ein Gemisch aus Propionsäure und Cyclohexanon abgezogen. Dieses höher als Propionsäure siedende Gemisch wird vorteilhafterweise über (13) der Verfahrensstufe (a) — der Umsetzung von Cyclohexanon mit der organischen Lösung der Perpropionsäure — wiederzugeführt.

Diese Kreisführung eines aus Propionsäure und Cyclohexanon bestehenden Gemisches stellt eine besondere Ausführungsform des erfindungsgemässen Verfahrens dar, die sich von grossem Vorteil erweist, nicht nur für die Durchführung des Verfahrensschrittes (a), sondern auch für die sich anschliessenden Destillationsschritte (b), (c) und (d).

Propionsäure und Cyclohexanon bilden unter bestimmten Bedingungen ein Azeotrop, d.h. ein konstant siedendes Gemisch. Dieses Azeotrop aus Propionsäure und Cyclohexanon wird an dieser Stelle erstmals beschrieben. Es siedet beispielsweise bei einem Druck von 133 mbar bei 92,2°C und besitzt die folgende Zusammensetzung: 27,1 Gew.-% Propionsäure und 72,9 Gew.-% Cyclohexanon.

Die Entnahme des Azeotrops oder eines in der Nähe des azeotropen Punktes sich befindlichen Gemisches aus Propionsäure und Cyclohexanon aus Kolonne (11) erfolgt im allgemeinen 1-10 theoretische Trennstufen oberhalb des Sumpfs der Kolonne als Strom (13). Es ist aber auch möglich, das Azeotrop oder ein Propionsäure und Cyclohexanon enthaltendes Gemisch als Sumpfprodukt aus (11) abzuziehen. Bevorzugt wird die Entnahme des Produktstromes (13) jedoch an eine Stelle gelegt, die sich 1-5 theoretische Trennstufen oberhalb des Sumpfes der Kolonne (11) befindet, ganz besonders bevorzugt 1-3 theoretische Böden oberhalb des Sumpfes.

Im allgemeinen wählt man die Betriebsbedindungen der Kolonne (11), insbesondere den Druck sowie weitere Parameter des erfindungsgemässen Verfahrens derart, dass das aus Propionsäure und Cyclohexanon bestehende Gemisch 0,5-50, bevorzugt 15-40 Gew.-% Propionsäure enthält. Es ist aber auch möglich, über (13) ein Gemisch abzuziehen und der Stufe (a) wiederzuzuführen, das mehr als 50 Gew.-% oder weniger als 15 Gew.-% Propionsäure enthält. Vorteilhaft ist es jedoch, wenn die Zusammensetzung des Produktstromes (13) in der Nähe der Zusammensetzung des Azeotrops liegt. Bevorzugt wählt man daher eine Konzentration an Propionsäure in (13), die im Bereich von 20-45 Gew.-%, ganz besonders bevorzugt im Bereich von 25-35 Gew.-% liegt. Man kann natürlich auch genau die azeotrope Zusammenstzung, die beispelsweise bei einem Druck von 133 mb 27 Gew.-% Propionsäure beträgt, im Strom (13) einstellen. Jedoch zeigt die Erfahrung bei kontinuierlichem Betrieb der Kolonne (11), dass es für ein störungsfreies Betreiben günstiger ist, die Konzentration an Propionsäure in (13) etwas oberhalb des der azeotropen Zusammensetzung entsprechenden Gehaltes zu wählen, beispielsweise bei 33 Gew.-% Propionsäure.

Das in geringer Menge anfallende Sumpfprodukt der Kolonne (11) wird über (14) der Kolonne (7) zugeführt, wo es an einer Stelle eingeführt wird, die sich oberhalb der im Seitenstrom erfolgenden Entnahme des ε-Caprolactons (Strom [9]) befindet. Der Produktstrom (14) enthält im wesentlichen Propionsäure und Cyclohexanon. Daneben können geringe Mengen an ε-Caprolacton und 2-Cyclohexylidencyclohexanon vorhanden sein.

Als Kopfprodukt (12) der Destillationseinheit (11) wird Propionsäure gewonnen, die weniger als 2 Gew.-% Cyclohexanon, bevorzugt weniger als 1 Gew.-%, ganz besonders bevorzugt nicht mehr als 0,5 Gew.-% Cyclohexanon enthält. In der über (12) entnommenen Propionsäure können geringe Mengen an Wasser enthalten sein. Im allgemeinen beträgt der Gehalt von $H_2O$ in (12) jedoch nicht mehr als 0,2 Gew.-%.

Kolonne (11) wird man im allgemeinen bei Drücken zwischen 0,01 und 1,5 bar betreiben. Bevorzugt erfolgt die Destillation in (11) im Druckbereich von 0,05 - 1,0 bar, ganz besonders bevorzugt im Bereich von 0,05 - 0,5 bar. Es ist von Vorteil, den Druck in der Kolonne (11) so zu wählen, dass sich im Bereich der Entnahme von Strom (13) eine Temperatur einstellt, die zwischen 80°C und 120°C liegt.

Das Rücklaufverhältnis, bei dem Kolonne (11) betrieben wird, kann in weiten Grenzen variiert werden, jedoch sind Rücklaufverhältnisse von 3 - 15 : 1 im allgemeinen für das Erreichen von weniger als 2 Gew.-% Cyclohexanon im Strom (12) geeignet.

Bevorzugt wird ein Rücklaufverhältnis gewählt, das bei 5 - 10 : 1 liegt. Die sich so ergebenden Mengen an Rücklauf auf die Kolonne sind dann ausreichend, um den Gehalt an Cyclohexanon in (12) auf Werte unter 0,5 Gew.-% zu halten, sofern die Kolonne (11) insgesamt 50-70 theoretische Trennstufen besitzt, wobei in vorteilhafter Weise der Einlauf des Produktstromes (10) dann an einer Stelle erfolgt, die sich 20 - 30 theoretische Trennstufen unterhalb des Kopfes der Kolonne befindet.

Nach dem erfindungsgemässen Verfahren kann ε-Caprolacton in Ausbeuten von über 96%, bezogen auf eingesetzte Perpropionsäure, und mindestens 95%, bezogen auf eingesetztes Cyclohexanon, hergestellt werden. Diese Zahlen verdeutlichen die Vorteile des erfindungsgemässen Verfahrens gegenüber allen bisherigen Verfahren. Das erfindungsgemäss hergestellte ε-Caprolacton weist daneben eine hohe Reinheit auf.

Beispiel 1 (vgl. Fig. 3)

Die Umsetzung zwischen der Lösung von Perpropionsäure in 1,2-Dichlorpropan und dem Cyclohexanon wird kontinuerlich in einer fünfstufigen Kesselkaskade (1) vorgenommen. Jeder Kessel hat ein Volumen von etwa 0,5 l und ist mit einem Rührer versehen. Die ersten drei Kessel werden bei einer Temperatur von 45°C betrieben, während die beiden nachfolgenden jeweils auf 55°C gehalten werden. Der Kesselkaskade nachgeschaltet ist ein Verweilzeitrohr, das einen Inhalt von ca. 1,8 l besitzt. In diesem Verweilzeitrohr wird die Temperatur bei etwa 57-57°C gehalten.

Bei kontinuierlichem Betrieb werden dem beschriebenen Reaktionssystem über den ersten Kessel stündlich 1910 ml einer 16,5 gew.%igen Lösung von Perpropionsäure in 1,2-Dichlorpropan (Strom 2) und über Leitung (3) 348 g Cyclohexanon, das eine Reinheit von über 99,5 Gew.-% besitzt, zugeführt. Die Lösung der Perpropionsäure enthält 11,2 Gew.-% Propionsäure, 0,2 Gew.-% Wasserstoffperoxid sowie etwa 0,1 Gew.-% Wasser. Der Gehalt an Schwefelsäure liegt unter 100 ppm. Neben der Perpropionsäure und dem Cyclohexanon (348 g) werden über Leitung (4) pro Stunde 508 g eines Gemisches in (1) eingespeist, das neben 33,27 Gew.-% Propionsäure, 66,54 Gew.% Cyclohexanon und 0,19 Gew.-% ε-Caprolacton enthält. Das Einsatz-Molverhältnis von Cyclohexanon zu Perpropionsäure beträgt somit 2 : 1. Das Reaktionssystem (1) wird bei einem Druck von 1,1 bar betrieben. Über Leitung (5) werden in den ersten Kessel geringe Mengen an Stickstoff (10 l/h) eingegeben. Der Perpropionsäure-Umsatz wird hinter dem Verweilzeitrohr zu 99,8% bestimmt. Nach dem Passieren des Verweilzeitrohres wird das Reaktionsgemisch im Behälter (6) entspannt, wobei neben dem Stickstoff auch geringe Mengen an Sauerstoff und $CO_2$ freigesetzt werden. Der Abgasstrom wird über Leitung (7) abgeführt.

Das hinter (6) in einer Menge von etwa 2,76 kg pro Stunde anfallende Reaktionsgemisch enthält neben dem 1,2-Dichlorpropan 14,0 Gew.-% ε-Caprolacton, 23,25 Gew.-% Propionsäure, das überschüssige Cyclohexanon und 0,13 Gew.-% Wasser sowie etwa 0,5 - 0,55 Gew.-% hochsiedende Anteile. Das Reaktionsgemisch wird über Leitung (8) der Kolonne (9) zugeführt, wo das 1,2-Dichlorpropan als Kopfprodukt wiedergewonnen wird. Die Kolonne (9) hat einen Durchmesser von 50 mm und besitzt im Abtreiberteil 10 Glockenböden sowie im Verstärkerteil 15 Glokkenböden. Sie ist mit einem Umlaufverdampfer als Beheizungssystem ausgestattet und wird bei einem Druck von 450 mbar betrieben. Das nach Kondensation im Kühler (10) anfallende Dichlorpropan wird aufgeteilt, so dass stündlich etwa 2,4 l auf den Kopf der Kolonne gegeben werden, während über Leitung (11) etwa 1,375 g Dichlorpropan pro Stunde abgezogen werden. Diese über (11) erhaltene 1,2-Dichlorpropan wird der Gewinnung der Perpropionsäure-Lösung wiederzugeführt.

Produktstrom (11) enthält etwa 0,2 - 0,3 Gew.-% Wasser sowie Spuren von Propionsäure. Die Temperatur am Kopf der Kolonne beträgt ca. 80°C. Im Sumpfprodukt stellt sich eine Temperatur ein, die zwischen 125 und 130°C liegt.

Das vom Lösungsmittel Dichlorpropan befreite Produktgemisch wird aus dem Sumpf der Kolonne (9) abgezogen und über Leitung (12) der Kolonne (13) zugeführt. Diese Kolonne (13) besitzt insgesamt 30 praktische Böden in Form von Glockenböden und hat einen Durchmesser von 50 mm. Die Eingabe des Stromes (12), der 28 Gew.-% ε-Caprolacton enthält, in die Kolonne (13) erfolgt auf dem 20. Boden (von unten gezählt). Die Abnahme des Caprolactons im Seitenstrom wiederum wird in Höhe des 10. Bodens dampfförmig vorgenommen. Die Kolonne wird bei einem Druck von 30 mbar und mit einem Rücklaufverhältnis von Rücklauf (14) zu Entnahme (15) von 1 : 1 betrieben. Die Beheizung der Kolonne (13) erfolgt ebenfalls mit einem Umlaufverdampfer, dem aber ein Dünnschichtverdampfer nachgeschaltet ist. Die in die Kolonne eingebrachten hochsiedenden Anteile, die im wesentlichen Adipinsäure neben polymerem Caprolacton enthalten, werden über Leitung (16) als Ablauf des Dünnschichters aus dem Verfahren ausgetragen. Die Menge beträgt stündlich nur etwa 15 g. Im Sumpf der Kolonne (13) wird eine Temperatur von 138-142°C festgestellt, die Kopftemperatur beträgt 60°C. Das über (17) als Seitenstrom aus (13) ausgetragene ε-Caprolacton

besitzt eine Reinheit von 99,7 - 99,9% und fällt in einer Menge von 385 g/h an.

Als Kopfprodukt der Kolonne (13) wird ein Gemisch aus Propionsäure und Cyclohexanon gewonnen, das über Leitung (15) in die Kolonne (18) eingegeben wird. Die Zusammensetzung des Stromes (15) beträgt im Mittel 65,45 Gew.-% Propionsäure, 34,45 Gew.-% Cyclohexanon und etwa 0,1 Gew.-% Caprolacton.

Die Kolonne (18) ist mit 50 praktischen Böden ausgerüstet und wird mit einem Umlaufverdampfer beheizt. Die Eingabe (15) wird auf dem 20. Boden, von unten aus gerechnet, vorgenommen. Die Kolonne (18) wird bei einem Kopfdruck von 130 mbar und einer Kopftemperatur von 85°C betrieben. Das Rücklaufverhältnis wird auf 6 : 1 eingestellt. Die Seitenstromentnahme für den Produktstrom (4) befindet sich in Höhe des 5. Bodens.

Als Destillat wird aus (18) über Leitung (19) Propionsäure in einer Menge von stündlich etwa 470-475 ml abgezogen und der Herstellung der Lösung der Perpropionsäure wiederzugeführt. In dieser Propionsäure sind nur Spuren von Cyclohexanon nachweisbar. Aus dem Sumpf der Kolonne (18) werden über (20) geringste Mengen an hochsiedenden Anteilen abgezogen, die man der Kolonne (13) zuführt.

Die Selektivität für ε-Caprolacton beträgt, bezofen auf die in das Reaktionssystem (1) eingesetzte Perpropionsäure 96,5% und bezogen auf verbrauchtes, d.h. als Frisch-Cyclohexanon (3) in (1) eingespeistes, Cyclohexanon, 95,2%.

Beispiel 2 (vgl. Fig. 2)

In einem, mit einem Doppelmantel versehenen und aus dem Werkstoff mit der DIN-Bezeichnung 1 4571 gefertigten Rohrreaktor mit einem Volumen von ca. 175 l wird an dem einen Ende stündlich 16,58 kg (168,9 Mol) frisches Cyclohexanon (14) und der Sumpfabzug (13), der aus 16,4 kg (167,1 Mol) Cyclohexanon und 8,33 kg (112,5 Mol) Propionsäure besteht, eingespeist. Gleichzeitig werden an derselben Öffnung des Reaktors kontinuierlich während einer Stunde 76,64 kg einer Lösung von Perpropionsäure in Benzol eingespeist (4). Eine genaue Analyse zeigt, dass in dieser Lösung pro Stunde 9,74 kg (131,5 Mol) Propionsäure; 15,21 kg (168,9 Mol) Perpropionsäure; 0,068 kg (2,0 Mol) Wasserstoffperoxid; 0,056 kg (3,1 Mol) Wasser und 51,57 kg (660,2 Mol) Benzol enthalten sind. Durch einen äusseren Wasserkreislauf im Doppelmantel wird im Reaktionsrohr eine Temperatur von ca. 50°C eingestellt. Am Ende des Reaktionsrohres sind nur noch Spuren an Perpropionsäure nachzuweisen.

Das ausragierte Gemisch wird nun einer Destillationseinheit (1) zugeführt, die aus einer insgesamt 5 m hohen Füllkörper-Kolonne mit einem Durchmesser von 0,2 m und einer Füllung aus Metall-Pallringen besteht. Die Einführung dieses Stromes (2) erfolgt etwa 6 theoretische Böden unterhalb des Kolonnenkopfes.

Über eine Vakuumpumpe wird ein Kopfdruck in dieser Kolonne von 0,43 bar eingestellt. Die Beheizung der Kolonne erfolgt durch einen Umlaufverdampfer.

Die aufsteigenden Dämpfe werden am Kopf der Kolonne total kondensiert und die flüssige Phase in die Ströme (3) und (5) aufgeteilt. Über den Strom (5) werden stündlich 46,5 kg als Rücklauf auf die Kolonne gegeben und über den Strom (3) stündlich 51,66 kg als Destillatentnahme entnommen, d.h. es wird ein Rücklaufverhältnis von R : E = 0,9 eingestellt. Eine Analyse des Stromes (3) zeigt, dass er zu 99,8% aus Benzol und zu 0,2% aus Wasser besteht.

Die restlichen Teile des Stromes (2) werden aus dem Sumpf der Kolonne als Strom (6) abgezogen und der Destillationseinheit (7) zugeführt. Die Destillationseinheit (7) besteht aus einer insgesamt 7 m hohen Füllkörperkolonne, die einen Durchmesser von 0,3 m besitzt und mit Metall-Pallringen als Füllkörper ausgestattet ist.

Der Einlauf des Stromes (6) in die Kolonne (7) erfolgt etwa 8 theoretische Böden unterhalb des Kolonnenkopfes. Wiederum ca. 8 theoretische Böden unterhalb der Einlaufstelle befindet sich eine Öffnung in der Kolonne, um einen dampfförmigen Seitenstrom entnehmen zu können. Der Sumpf der Kolonne befindet sich etwa 5 theoretische Stufen unterhalb dieser Seitenentnahme.

Die Heizung der Kolonne erfolgt durch einen Dünnschichtverdampfer. Über ein Vakuumsystem wird ein Kopfdruck in der Kolonne (7) von 33 mbar eingestellt. Die aufsteigenden Dämpfe werden am Kopf der Kolonne durch einen Dephlegmator geleitet, der durch Wasserkühlung so eingestellt ist, dass ein Rücklaufverhältnis von 0,8 erhalten wird. Die durch den Dephlegmator steigenden Dämpfe werden in einem weiteren Kondensator total kondensiert und kontinuierlich als Strom (10) der Destillationseinheit (11) zugeführt.

In Seitenstrom (9) wird gleichzeitig kontinuierlich dampfförmiges ε-Caprolacton abgezogen. Ein dort angebrachtes Thermometer zeigt eine Dampftemperatur von 135°C. Das dampfförmige Caprolacton wird kondensiert und gewogen. Es ergeben sich so stündlich 18,80 kg (164,7 Mol) ε-Caprolacton.

Durch den Dünnschichtverdampfer der Kolonne (7) laufen stündlich 0,51 kg höhersiedende Verbindungen, die nicht verdampft und aus dem Verfahren über (8) ausgeschleust werden. Das Destillat der Kolonne (7), der Strom (10), wird einer weiteren Destillationseinheit (11) zugeführt. Diese Destillationseinheit besteht aus einer insgesamt 12 m hohen Füllkörperkolonne mit einem Durchmesser von 0,3 m und Metall-Pallringen als Füllung. Über eine Vakuumpumpe wird in der Kolonne ein Kopfdruck von 135 mbar eingestellt. Die Beheizung der Kolonne erfolgt durch einen Fallfilmverdampfer.

Die aufsteigenden Dämpfe werden am Kopf der Kolonne total kondensiert und ein Teil des Kondensats als Rücklauf auf die Kolonne gegeben. Es wird ein Rücklaufverhältnis von 6 : 1 eingestellt. Als Destillatentnahme werden pro Stunde 22,25 kg flüssige Phase (12) abgezogen. Eine G.C. Analyse zeigt, dass dieses Produkt zu 99,95% aus Propionsäure besteht und nur Spuren von Cyclohexanon und Wasser enthalten sind.

Aus dem Sumpf dieser Kolonne wird kontinuierlich der Strom (13) abgezogen und in das Reaktionssystem gefördert. Eine Analyse zeigt, dass sich dieser Strom aus 16,4 kg (167,1 Mol) Cyclohexanon pro Stunde und 8,33 kg (112,5 Mol) Propionsäure pro Stunde zusammensetzt.

Aus der im Strom (9) erhaltenen Menge von 18,80 kg Caprolacton/h errechnet sich eine Ausbeute von 97,5%, bezogen auf Perpropionsäure in (4), und ebenfalls von 97,5%, bezogen auf Cyclohexanon, in (14).

**Patentansprüche**

1. Verfahren zur Herstellung von ε-Caprolacton durch Umsetzung von Cyclohexanon mit Perpropionsäure, dadurch gekennzeichnet, dass man

a) eine Lösung von Perpropionsäure in einem organischen Lösungsmittel, das mindestens 15°C unterhalb des Siedepunktes von Propionsäure siedet, mit Cyclohexanon im Molverhältnis Cyclohexanon : Perpropionsäure wie 1,1 - 5 : 1 bei Temperaturen von 10 - 80°C umsetzt,

b) das erhaltene, im wesentlichen aus ε-Caprolacton, Cyclohexanon, Propionsäure und organischem Lösungsmittel bestehende Reaktionsgemisch einer ersten Destillationseinheit zuführt, wo das organische Lösungsmittel für die Perpropionsäure als Destillat wiedergewonnen wird,

c) das Sumpfprodukt der ersten Destillationseinheit in eine zweite Destillationseinheit eingibt, wo die Propionsäure zusammen mit in Stufe (a) nicht umgesetztem Cyclohexanon als Kopfprodukt gewonnen wird und ε-Caprolacton sowie gegebenenfalls hochsiedende Anteile getrennt voneinander unterhalb des Einlaufes aus dieser zweiten Destillationseinheit abgezogen werden und

d) das aus Propionsäure und Cyclohexanon bestehende Destillat der zweiten Destillationseinheit in eine dritte Destillationseinheit überführt, wo ein aus Propionsäure und Cyclohexanon bestehendes Gemisch erhalten und als Destillat Propionsäure wiedergewonnen wird.

2. Verfahren nach Anspruch 1, dadurch gekennzeichnet, dass man das in Stufe (d) anfallende, Cyclohexanon und Propionsäure enthaltende Gemisch der Umsetzungsstufe gemäss (a) wiederzugeführt.

3. Verfahren nach Anspruch 1 und 2, dadurch gekennzeichnet, dass das in Stufe (d) anfallende Gemisch aus Propionsäure und Cyclohexanon 0,5-50 Gew.-% Propionsäure enthält.

4. Verfahren nach Anspruch 1 bis 3, dadurch gekennzeichnet, dass man in der für die Umsetzung gemäss (a) verwendeten organischen Lösung der Perpropionsäure Gehalte von weniger als 0,5 Gew.-% Wasser, weniger als 0,25 Gew.-% Wasserstoffperoxid und weniger als 100 ppm starker Säure einstellt.

5. Verfahren nach Anspruch 1 bis 4, dadurch gekennzeichnet, dass man als organische Lösungsmittel für die Perpropionsäure Benzol, 1,2-Dichlorpropan, Trichloräthylen, Tetrachloräthylen, Methylenchlorid, 1,2-Dichloräthan, Essigsäurepropylester, Propylpropionat oder Diisopropyläther verwendet.

6. Verfahren nach Anspruch 1 bis 5, dadurch gekennzeichnet, dass man als organische Lösung der Perpropionsäure eine Lösung einsetzt, die durch Extraktion eines Perpropionsäure, Wasser, sauren Katalysator, Propionsäure und Wasserstoffperoxid enthaltenden Gemisches mit dem organischen Lösungsmittel gewonnen worden ist.

7. Verfahren nach Anspruch 1 bis 6, dadurch gekennzeichnet, dass man die Entnahme des ε-Caprolactons aus der zweiten Destillationseinheit 1 bis 15 theoretische Trennstufen oberhalb des Sumpfes vornimmt und die gegebenenfalls mit in die zweite Destillationseinheit eingebrachten höhersiedenden Anteile als Sumpfprodukt aus dieser Destillationseinheit austrägt.

8. Verfahren nach Anspruch 1 bis 7, dadurch gekennzeichnet, dass man die Entnahme des aus Propionsäure und Cyclohexanon bestehenden Gemisches aus der dritten Destillationseinheit 1 bis 3 theoretische Trennstufen oberhalb des Sumpfes vornimmt.

9. Verfahren nach Anspruch 1 bis 8, dadurch gekennzeichnet, dass man in der als Destillat in der dritten Destillationseinheit gewonnenen Propionsäure einen Gehalt von weniger als 0,5 Gew.-% Cyclohexanon einstellt und dieses Destillat der Gewinnung der Perpropionsäure wiederzuführt.

**Claims**

1. Process for the preparation of ε-caprolactone by reacting cyclohexanone with perpropionic acid, characterised in that a) a solution of perpropionic acid in an organic solvent which has a boiling point at least 15°C below that of propionic acid is reacted with cyclohexanone in a molar ratio of cyclohexanone : perpropionic acid of 1.1 - 5 : 1 at temperatures of 10-80°C, b) the resulting reaction mixture essentially consisting of ε-caprolactone, cyclohexanone, propionic acid and organic solvent is passed to a first distillation unit where the organic solvent for the perpropionic acid is recovered as the distillate, c) the bottom product from the first distillation unit is introduced into a second distillation unit where the propionic acid and cyclohexanone which hat not been reacted in stage (a) are obtained as the top product and ε-caprolactone and any high-boiling constituents are removed, separately from one another, below the inlet from this second distillation unit and d) the distillate, consisting of propionic acid

and cyclohexanone, from the second distillation unit is transferred to a third distillation unit where a mixture consistintg of propionic acid and cyclohexanone is obtained and propionic acid is recovered as the distillate.

2. Process according to Claim 1, characterised in that the mixture containing cyclohexanone and propionic acid which is obtained in stage (d) is recycled to reaction stage (a).

3. Process according to Claims 1 and 2, characterised in that the mixture of propionic acid and cyclohexanone obtained in stage (d) contains 0.5-50% by weight of propionic acid.

4. Process according to Claims 1 to 3, characterised in that a water content of less than 0.5% by weight, a hydrogen peroxide content of less than 0.25% by weight and a strong acid content of less than 100 ppm is established in the organic solution of perpropionic acid used for the reaction in stage (a).

5. Process according to Claims 1 to 4, characterised in that benzene, 1,2-dichloropropane, trichloroethylene, tetrachloroethylene, methylene chloride, 1,2-dichloroethane, propyl acetate, propyl propionate or diisopropyl ether is used as the organic solvent for the perpropionic acid.

6. Process according to Claims 1 to 5, characterised in that a solution which has been obtained by extraction of a mixture containing perpropionic acid, water, an acid catalyst, propionic acid and hydrogen peroxide with the organic solvent is used as the organic solution of perpropionic acid.

7. Process according to Claims 1 to 6, characterised in that the ε-caprolactone is taken off from the second distillation unit 1 to 15 theoretical plates above the bottom and the higher-boilling constituents which may have been introduced into the second distillation unit are discharged from this distillation unit as the bottom product.

8. Process according to Claims 1 to 7, characterised in that the mixture consisting of propionic acid and cyclohexanone is taken off from the third distillation unit 1 to 3 theoretical plates above the bottom.

9. Process according to Claims 1 to 8, characterised in that a cyclohexanone content of less than 0.5% by weight is established in the propionic acid obtained as the distillate in the third dstillation unit, and this distillate is recycled to the production of the perpropionic acid.

**Revendications**

1. Procédé pour la fabrication d'ε-caprolactone par réaction de la cyclohexanone avec l'acide perpropionique, caractérisé en ce que

a) on fait réagir une solution d'acide perpropionique, dans un solvant organique qui bout au moins 15°C au-dessous du point d'ébullition de l'acide propionique, avec la cyclohexanone dans un rapport molaire cyclohexanone/acide perpropionique de 1,1 - 5 : 1 à des températures de 10-80°C,

b) en envoie le mélange de réaction obtenu, qui consiste essentiellement en ε-caprolactone, cyclohexanone, acide propionique et solvant organique, à une première unité de distillation, où le solvant organique pour l'acide perpropionique est, récupéré come distillat,

c) on introduit le résidu de la première unité de distillation dans une seconde unité de distillation, où l'on récupère comme produit de tête l'acide propionique simultanément avec la cyclohexanone non transformée dans l'étape a), et l'on retire de cette seconde unité de distillation l'ε-caprolactone, ainsi éventuellement que les fractions à haut point d'ébullition séparément l'une de l'autre au-dessous du point d'introduction, et

d) on envoie le distillat de la seconde unité de distillation, consistant en acide propionique et cyclohexanone, dans une troisième unité de distillation, où l'on obtient un mélange consistant en acide propionique et cyclohexanone, et on récupère de l'acide propionique comme distillat.

2. Procédé selon la revendication 1, caractérisé en ce que l'on renvoie à l'étape de réaction selon a) le mélange contenant la cyclohexanone et l'acide propionique se formant dans l'étape d).

3. Procédé selon les revendications 1 et 2, caractérisé en ce que le mélange d'acide propionique et de cyclohexanone se formant dans l'étape d) contient 0,5-50% en poids d'acide propionique.

4. Procédé selon les revendications 1 à 3, carectérisé en ce que l'on ajuste dans la solution organique d'acide perpropionique utilisée pour la réaction selon a) des teneurs de moins de 0,5% en poids d'eau, moins de 0,25% en poids de peroxyde d'hydrogène et moins de 100 ppm d'acide fort.

5. Procédé selon les revendications 1 à 4, caractérisé en ce que l'on utilise comme solvant organique pour l'acide perpropionique le benzène, le 1,2-dichloropropane, le trichloroéthylène, le tétrachloroéthylène, le chlorure de méthylène, le 1,2-dichloroéthane, l'acétate de propyle, le propionate de propyle ou l'éther diisopropylique.

6. Procédé selon les revendications 1 à 5, caractérisé en ce que l'on utilise comme solution organique de l'acide perpropionique une solution qui a été obtenu par extraction par le solvant organique d'un mélange contenant de l'acide perpropionique, de l'eau, un catalyseur acide, de l'acide propionique et du peroxyde d'hydrogène.

7. Procédé selon les revendications 1 à 6, caractérisé en ce que l'on effectue le prélèvement de l'ε-caprolactone de la seconde unité de distillation entre 1 et 15 plateaux théoriques de séparation au-dessus du fond et on retire comme résidu de cette unité de distillation les fractions à haut point d'ébullition éventuellement introduites simultanément dans la seconde unité de distillation.

8. Procédé selon les revendications 1 à 7, caractérisé en ce que l'on prélève de la troisième unité de distillation le mélange consistant en acide propionique et cyclohexanone entre 1 et 3 plateaux théoriques de séparation au-dessus du fond.

9. Procédé selon les revendications 1 à 8, caractérisé en ce que l'on ajuste une teneur de moins de 0,5% en poids de cyclohexanone dans l'acide propionique obtenu comme distillat dans la troisième unité de distillation et on renvoie ce distillat à la récupération de l'acide perpropionique.

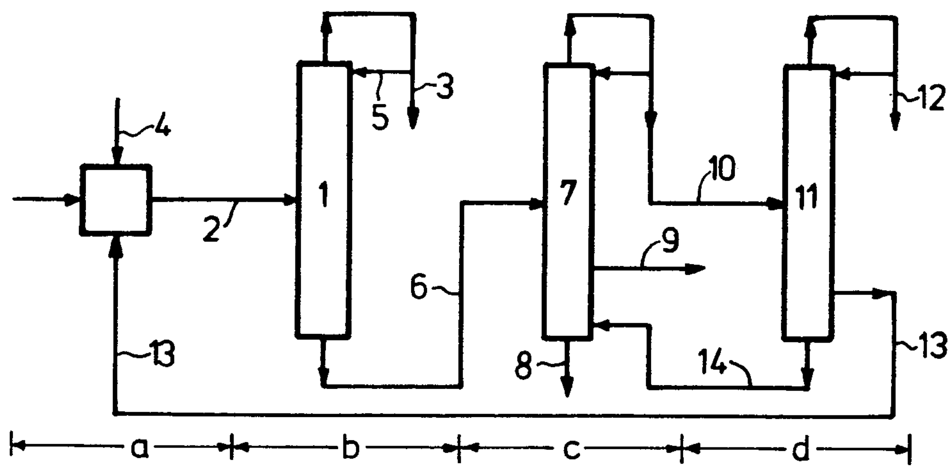


FIG. 1

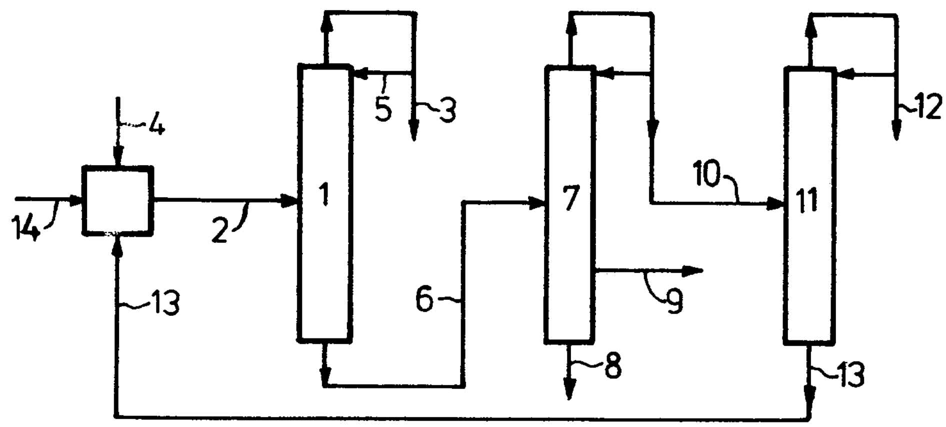


FIG. 2

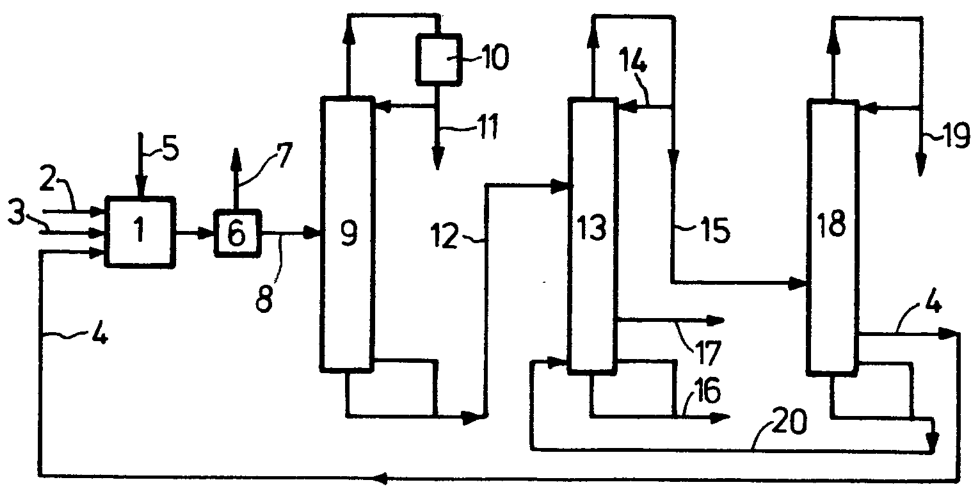


FIG. 3